Europäisches Patentamt

European Patent Office

Office européen des brevets

(19)

(11) Numéro de publication : **0 657 409 A1**

## (12) DEMANDE DE BREVET EUROPEEN

(21) Numéro de dépôt : **94402780.4**

(51) Int. Cl.⁶ : **C07C 17/20, C07C 19/08**

(22) Date de dépôt : **05.12.94**

(30) Priorité : **09.12.93 FR 9314780**

(43) Date de publication de la demande :
**14.06.95 Bulletin 95/24**

(84) Etats contractants désignés :
**DE ES FR GB GR IT NL**

(71) Demandeur : **ELF ATOCHEM S.A.**
**4 & 8, Cours Michelet**
**La Défense 10**
**F-92800 Puteaux (FR)**

(72) Inventeur : **Garcia, François**
**50 Chemin du Bois**
**F-69530 Brignais (FR)**
Inventeur : **Lacroix, Eric**
**Le bourg**
**F-69480 Amberieux d'Azergues (FR)**
Inventeur : **Lerch, Alain**
**2 Rue Barrau**
**F-31400 Toulouse (FR)**
Inventeur : **Rousset, Abel**
**16 Rue Jean Moulin**
**F-31520 Ramonville (FR)**

(74) Mandataire : **Leboulenger, Jean et al**
**Elf Atochem S.A.**
**Département Propriété Industrielle, Cedex 42 -**
**La Défense 10**
**F-92091 Paris la Défense (FR)**

(54) **Fluoration catalytique d'hydrocarbures halogénés en phase gazeuse.**

(57)     L'invention concerne la fluoration catalytique d'hydrocarbures halogénés en phase gazeuse au moyen d'acide fluorhydrique.
    On utilise un catalyseur mixte à base de dérivés du chrome et du vanadium, ces deux éléments étant majoritairement à l'état d'oxydation III.

EP 0 657 409 A1

Jouve, 18, rue Saint-Denis, 75001 PARIS

La présente invention concerne la fabrication d'hydrocarbures fluorés par fluoration catalytique d'hydrocarbures halogénés en phase gazeuse au moyen d'acide fluorhydrique et a plus particulièrement pour objet l'emploi de catalyseurs à base de chrome et de vanadium.

Les recherches intenses menées sur les substituts aux chlorofluorocarbures (CFC) s'orientent, entre autres, vers la synthèse d'hydrohalogénoalcanes fluorés. Certaines étapes de cette synthèse peuvent être réalisées par fluoration catalytique en phase gazeuse hétérogène au moyen d'acide fluorhydrique.

Ces hydrohalogénoalcanes étant souvent des composés plus complexes que les CFC et, surtout, chimiquement plus réactifs que ces derniers, leur synthèse nécessite la mise au point de catalyseurs plus actifs et plus sélectifs.

De très nombreux composés métalliques (par exemple chrome, cobalt, nickel, manganèse, magnésium, vanadium, cuivre,...) présentent un effet catalytique pour ces réactions de fluoration. Les catalyseurs proposés dans la littérature sont soit massiques, soit supportés, les supports couramment utilisés étant principalement le charbon, l'alumine (transformée partiellement en AlF₃ après fluoration) ou le trifluorure d'aluminium.

L'association du chrome et du vanadium est connue pour la préparation de catalyseurs d'oxydation. Ainsi, le brevet EP 002682 revendique un catalyseur massique à base d'oxydes de vanadium et de chrome, pour l'oxydation d'hydrocarbures aliphatiques en acides carboxyliques.

Dans le domaine de la fluoration, peu de documents citent l'emploi du vanadium dans des catalyseurs de fluoration.

A ce titre, le brevet EP 295885 décrit la synthèse du F134a (1,1,1,2-tétrafluoroéthane) par fluoration en phase gazeuse d'une oléfine halogénée sur un catalyseur à base de chrome hexavalent et d'un métal de transition supportés sur alumine. Le métal de transition plus particulièrement préféré est le titane. Le vanadium est cité mais n'est pas exemplifié. Partant d'alumine solide, l'imprégnation est assurée par des solutions aqueuses d'oxyde de chrome VI et de sels des métaux de transition ; en fin de préparation, le chrome est toujours à l'état VI et l'autre métal est sous une forme oxydée (état d'oxydation IV pour le titane).

Par ailleurs, le brevet EP 203807 décrit la préparation d'oléfines fluorées par transhalogénation en utilisant un catalyseur à base d'oxyde de chrome ou d'oxyde d'aluminium combiné à un composé d'autres éléments dont le vanadium. Les mixtes à base de vanadium ne sont pas exemplifiés et la réaction de base n'est pas une réaction de fluoration au sens de la présente invention.

Le vanadium est également utilisé pour la fluoration en phase liquide. Le brevet EP 503792 décrit la fluoration d'halogénoalcanes avec un fluorure de métal de transition tel que le pentafluorure de vanadium.

De même, Bardin et Coll. [J. Fluor. Chem. (1990) 49(3) 385-400] décrivent la fluoration d'oléfines (addition de deux atomes de fluor) en phase liquide. L'agent de fluoration utilisé est VF₅ synthétisé par fluoration directe du vanadium métal par le fluor.

Il a maintenant été trouvé que dans la fluoration en phase gazeuse, par HF, d'hydrocarbures halogénés, saturés ou oléfiniques, l'emploi de catalyseurs mixtes Cr-V dans lesquels le vanadium et le chrome se trouvent majoritairement à l'état d'oxydation III permet d'obtenir des performances catalytiques supérieures à celles obtenues avec le chrome seul ou avec le vanadium seul. Par ailleurs, l'utilisation de vanadium et de chrome à l'état III permet de réduire considérablement les pertes en vanadium en évitant la formation d'oxyfluorures ou de fluorures de vanadium ᵛ volatils. Les phases mixtes Cr-V à l'état d'oxydation III permettent donc d'améliorer les performances catalytiques, tout en limitant les pertes en espèces actives.

L'invention a donc pour objet un procédé de fluoration catalytique d'hydrocarbures halogénés en phase gazeuse au moyen d'acide fluorhydrique, caractérisé en ce qu'on utilise un catalyseur mixte à base de chrome et de vanadium, ces deux éléments étant majoritairement à l'état d'oxydation III.

Par "majoritairement" on entend qu'au moins 80 % (de préférence plus de 90 %) des éléments chrome et vanadium sont à l'état d'oxydation III.

Dans le catalyseur selon l'invention qui peut être massique ou supporté, le rapport atomique V/Cr peut aller de 0,05 à 5. Il est avantageusement compris entre 0,1 et 3,5, de préférence entre 0,15 et 3.

Le catalyseur à utiliser conformément à la présente invention peut être préparé à partir de précurseurs de chrome et de vanadium, par des procédés connus en soi pour l'obtention de catalyseurs massiques ou supportés.

Comme précurseurs du chrome on peut mentionner les oxydes, hydroxydes, halogénures, oxyhalogénures, nitrates, acétates, oxalates et sulfates de chrome, mais on peut aussi utiliser tout autre composé du chrome permettant de réaliser un mélange homogène en phase solide de chrome et de vanadium. Pour la préparation de catalyseurs massiques, les précurseurs préférés sont les sels de chrome comme les chlorures et plus particulièrement les sulfates, acétates et nitrates. Pour la préparation de catalyseurs supportés, on préfère utiliser le chlorure de chrome III ou l'anhydride chromique (CrO₃).

Les précurseurs du vanadium disponibles sont peu nombreux. On peut mentionner les chlorures, les acétylacétonates, les oxychlorures et les oxysulfates, le chlorure de vanadium III étant plus particulièrement préféré.

A titre d'exemple non limitatif, une méthode de préparation de catalyseurs massiques selon l'invention consiste en la neutralisation des composés précités du chrome III et du vanadium par une base (hydroxyde de sodium, ammoniaque, amines,...) ou bien en la réduction de $CrO_3$ en présence d'un composé du vanadium cité précédemment. Le précipité ou le gel obtenu est alors lavé puis séché avant d'être calciné à une température comprise entre 200 et 600°C.

Un catalyseur massique selon l'invention peut également être obtenu par décomposition thermique (200 à 450°C) d'oxalates de chrome et de vanadium, précipités en milieu organique (alcools, éthers, hydrocarbures chlorés,...)

Le catalyseur final peut être mis en forme selon toute technique connue de l'homme de l'art (pastillage, extrusion, granulation,...).Divers additifs peuvent être ajoutés à la préparation pour parfaire les propriétés physico-chimiques et catalytiques du produit final. On peut ainsi ajouter (% massique par rapport à la masse du catalyseur final) :

- 2 à 30 % de poudre de $Cr_2O_3$ ou de $Cr_2O_3$, $2H_2O$ séchée au préalable à 300°C, ou d'$Al_2O_3$, $xH_2O$ pour améliorer la tenue mécanique du catalyseur final,
- 0,1 à 5 % de graphite et/ou 0,1 à 10 % d'alcool polyvinylique pour faciliter la mise en forme par pastillage ou extrusion,
- 0,1 à 20 % de floculant tel que les polyacrylates ou polyacrylamides pour faciliter la filtration du gâteau récupéré après neutralisation.

Pour les catalyseurs supportés, le support utilisé doit être compatible avec les milieux contenant de l'HF. Conviennent les supports couramment utilisés tels que le trifluorure d'aluminium et les oxydes métalliques comme l'alumine, la magnésie, la zircone. Pour ces oxydes qui généralement se fluorent sous atmosphère d'HF (réaction exothermique génératrice d'eau), il est souvent préférable de les soumettre à des conditions fluorantes avant leur imprégnation par les composés du chrome et du vanadium. Cette imprégnation du support peut être réalisée, par exemple, au moyen d'une solution aqueuse de $CrCl_3$ et de $VCl_3$ ou au moyen de deux solutions aqueuses, l'une contenant $CrO_3$, l'autre contenant $VCl_3$ et un agent réducteur tel que le méthanol. Dans certains cas, le support (charbon) peut également intervenir comme agent réducteur. Dans un tel catalyseur supporté, la teneur totale en chrome et en vanadium peut aller de 0,5 à 25 % en poids, de préférence entre 2 et 15 %.

Les catalyseurs mixtes Cr-V selon l'invention peuvent être utilisés pour la fluoration par HF en phase gazeuse d'hydrocarbures halogénés, saturés ou oléfiniques. Ils conviennent particulièrement bien à la fluoration d'hydrocarbures halogénés conduisant à des composés fluorés en $C_1$ à $C_4$ contenant un ou plusieurs atomes d'hydrogène. Comme exemples d'hydrocarbures halogénés de départ, on peut mentionner, à titre non limitatif, les composés suivants: $CHCl_3$, $CH_2Cl_2$, $CCl_2=CHCl$, $CHCl_2-CClF_2$, $CHCl_2-CF_3$, $CHFCl-CF_3$, $CH_3-CCl_3$, $CH_3-CFCl_2$, $CH_3-CF_2Cl$, $CH_2Cl-CF_3$, $CH_3-CCl_2-CH_3$, $CCl_3-CF_2-CH_3$, $CCl_3-CF_2-CHCl_2$, $CCl_3-CF_2-CH_2Cl$, $CHCl_2-CHCl-CH_3$, $CH_2Cl-CHCl-CH_3$, ainsi que $CCl_2=CCl_2$; ce dernier composé ne contient pas d'hydrogène, mais l'addition d'HF conduit à des composés hydrohalogénés.

Pour travailler à l'activité optimum, le catalyseur nécessite un traitement à l'acide fluorhydrique, dilué ou non avec de l'azote. Bien que la présence de vanadium retarde la cristallisation, une telle activation peut générer localement des températures supérieures à 500°C. C'est pourquoi, il est recommandé de contrôler l'exothermicité de l'activation en jouant sur l'ajout d'un diluant de l'HF et en commençant ce traitement à basse température (150-250°C). Par contre, après passage des "vagues d'exothermicité" dans le lit catalytique, il est conseillé de monter progressivement la température pour atteindre un maximum de 350-450°C en fin d'activation.

Lorsque le catalyseur non activé contient une teneur importante en chrome et/ou en vanadium à un degré d'oxydation supérieur à trois, il convient de procéder à une étape de réduction (traitement thermique entre 200 et 400°C en atmosphère neutre ou réductrice) préalablement à l'activation pour éviter que cette dernière n'entraîne une perte importante de vanadium et/ou de chrome par formation d'espèces volatiles.

La température de fluoration des organiques dépend de la réaction étudiée et bien évidemment des produits de réaction désirés. Ainsi, pour une addition d'HF sur une double liaison ou une substitution partielle des atomes de chlore par le fluor, on travaille à des températures comprises entre 50 et 350°C. La substitution de la totalité des atomes de chlore nécessite généralement des températures comprises entre 300 et 500°C.

Le temps de contact dépend également de la réaction étudiée et des produits recherchés. Le plus souvent, il est compris entre 3 et 100 secondes ; cependant, pour obtenir un bon compromis entre taux de conversion et productivité, le temps de contact est avantageusement inférieur à 30 secondes.

Le rapport molaire HF/composé(s) organique(s) est également lié à la réaction étudiée. Il dépend entre autres de la stoechiométrie de la réaction. Dans la majorité des cas, il peut varier entre 1/1 et 20/1, mais là aussi, afin d'obtenir des productivités élevées, il est souvent inférieur à 10.

La pression de travail est de préférence comprise entre 1 et 20 bars absolus (0,1 à 2 MPa).

Les catalyseurs selon l'invention peuvent, suivant leur solidité mécanique, travailler en lit fixe ou en lit fluide. Les catalyseurs dont l'activité a chuté par suite d'un encrassement, peuvent être régénérés par balayage du catalyseur avec un composé susceptible d'oxyder et de transformer les produits (organiques, coke,...) déposés sur le catalyseur, en produits volatils. A ce titre, l'oxygène ou un mélange contenant de l'oxygène (air par exemple) convient parfaitement et permet de restaurer l'activité initiale du catalyseur.

Afin d'assurer la régénération du catalyseur sans induire une cristallisation du catalyseur ou une oxydation du vanadium et du chrome, il est recommandé d'effectuer ce traitement à une température n'excédant pas 350°C. Il convient donc de limiter l'exothermicité de cette "combustion" en contrôlant le débit d'oxygène (en début de régénération, faible débit d'oxygène dilué dans un inerte) de façon à maintenir une température inférieure à 350°C.

Pour maintenir l'activité du catalyseur, il est également possible d'effectuer la réaction de fluoration en présence d'oxygène introduit dans un rapport molaire $O_2$/composé organique pouvant aller de 0,001 à 0,05 et, de préférence, compris entre 0,005 et 0,03. Dans ce cas, il est recommandé de travailler à une température n'excédant pas 400°C pour éviter une oxydation du vanadium et du chrome.

Les exemples suivants illustrent l'invention sans la limiter.

## PREPARATION DES CATALYSEURS

### *Catalyseur 1*

On a dissout 20 g (0,05 mole) de nitrate de chrome nonahydraté $Cr(NO_3)_3$, $9H_2O$ et 1,57 g (0,01 mole) de trichlorure de vanadium $VCl_3$ dans 100 ml d'eau, puis la solution a été neutralisée jusqu'à pH 6,4 au moyen de 14 ml de $NH_4OH$ 14N. Le gel obtenu a été lavé à l'eau, puis filtré et séché à l'étuve pendant 14 heures à 120°C. La poudre ainsi obtenue a ensuite été calcinée sous azote à 350°C pendant 4 heures.

### *Catalyseur 2*

On a préparé à 60°C sous agitation une solution de 32 g d'oxalate d'ammonium monohydraté, 1,15 g de trichlorure de vanadium et 27 g de nitrate de chrome nonahydraté dans 75 ml d'eau. La solution limpide ainsi obtenue a été refroidie, puis versée rapidement dans un mélange de 375 ml d'éthanol et 375 ml d'éthylèneglycol.

Le complexe mixte $(NH_4)_3 [Cr_xV_{1-x}(C_2O_4)_3]$ ainsi obtenu a ensuite été décomposé sous air à 350°C, conduisant à un catalyseur amorphe d'oxydes de chrome et de vanadium.

### *Catalyseur 3*

Même préparation que le Catalyseur 2, mais avec une quantité double de $VCl_3$ (2,3 g) et une quantité moindre de $Cr(NO_3)_3$, $9H_2O$ (24 g).

### *Catalyseur 4 (Comparatif)*

Même préparation que le Catalyseur 1, mais en remplaçant $VCl_3$ par 1,73 g (0,01 mole) d'oxytrichlorure de vanadium $VOCl_3$.

### *Catalyseur 5*

Même préparation que le Catalyseur 1, mais en remplaçant $VCl_3$ par 1,73 g de $VOCl_3$ et en effectuant la calcination avec un mélange d'azote et d'hydrogène (rapport molaire $N_2/H_2$ : 95/5) pour réduire $V^V$ en $V^{III}$.

### *Catalyseur 6 (Comparatif)*

On a mélangé à 60°C sous agitation dans 75 ml d'eau 32 g d'oxalate d'ammonium monohydraté et 13 g de $VOCl_3$. Le complexe oxalique ainsi obtenu a ensuite été décomposé sous air à 350°C, conduisant à un catalyseur d'oxyde de vanadium $^V$.

### Catalyseur 7

Dans un évaporateur rotatif, on place 30 ml d'un support contenant en poids 73 % de fluorure d'aluminium et 27 % d'alumine, obtenu dans une étape précédente par fluoration d'alumine de grande surface (> 180 m²/g) en lit fluidisé vers 300°C à l'aide d'air et d'acide fluorhydrique (concentration volumique de 5 à 10 % de cet acide dans l'air).

On prépare par ailleurs deux solutions aqueuses séparées :

*(a)* solution chromique contenant :
- anhydride chromique (CrO₃) :        3,2 g
- eau :        3g

*(b)* Solution méthanolique contenant :
- trichlorure de vanadium (VCl₃) :        5 g
- méthanol :        3,8 g
- eau :        9g

Le mélange de ces deux solutions est ensuite introduit à température ambiante sous pression atmosphérique et en 45 minutes environ, sur le support en agitation. Le catalyseur est alors séché sous courant d'azote, en lit fluidisé, vers 110°C pendant 4 heures.

### Catalyseur 8 (Comparatif)

Même préparation que le catalyseur 7, mais sans réduction du chrome par le méthanol, en remplaçant la solution méthanolique de VCl₃ par une solution de 5 g de VCl₃ dans 11 ml d'eau.

### Catalyseur 9

Même préparation que le catalyseur 7, mais en imprégnant le support avec une seule solution contenant 8,5 g de chlorure de chrome III hexahydraté, 5 g de VCl₃ et 14 g d'eau.

### Catalyseur 10

Même préparation que le catalyseur 7, mais en remplaçant la solution méthanolique de VCl₃ par une solution contenant 5,4 g de VOCl3, 3,8 g de méthanol et 5 g d'eau.

### Catalyseur 11 (Comparatif)

Même préparation que le catalyseur 7, mais en remplaçant la solution méthanolique de VCl₃ par une solution de 5,4 g de VOCl₃ dans 8 g d'eau.

### Catalyseur 12 (Comparatif)

Même préparation que le catalyseur 7, mais sans vanadium en utilisant une solution chromique de 3,2 g de CrO₃ dans 5 g d'eau et une solution méthanolique contenant 3,8 g de méthanol dans 7 g d'eau.

### Catalyseur 13 (Comparatif)

Même préparation que le catalyseur 7, mais sans chrome en imprégnant le support avec une seule solution contenant 5 g de VCl₃, 10 g d'eau et 2 g de méthanol.

### FLUORATION DU F133a

Les performances des catalyseurs 1 à 13, ainsi que celles d'un catalyseur massique $Cr_2O_3$ amorphe (ci-après Catalyseur 14), ont été testées, après activation, dans la fluoration du 1-chloro-2,2,2-trifluoroéthane (F133a) à pression atmosphérique.

L'acide fluorhydrique utilisé est un produit commercial ne contenant que des traces d'eau et le F133a de départ est un produit pur à 99,9 %. Le réacteur utilisé est un tube en Inconel de 20 ml chauffé par un four tubulaire.

L'activation du catalyseur par HF est réalisée dans ce réacteur sur un échantillon de 15 ml. Après un séchage de 2 heures à 200°C sous azote (0,1 mole/heure), on ajoute progressivement de l'HF à cette même température. Après passage des pics d'exothermicité, le débit d'HF est augmenté pour atteindre 0,1 mole/heure, puis la température est portée à 350°C. Un palier de température est observé dans ces conditions pendant 10 heures.

Avant leur introduction dans le réacteur, les réactifs sont mélangés et chauffés à la température de réaction dans un préchauffeur en Inconel.

Après lavage à l'eau (pour éliminer les hydracides) et séchage sur $CaCl_2$, les produits de réaction sont analysés en ligne, par chromatographie en phase gazeuse.

Le Tableau I suivant indique les principales caractéristiques des catalyseurs activés, ainsi que le pourcentage de pertes en vanadium durant l'activation.

Les Tableaux II et III rassemblent les conditions opératoires de la fluoration du F133a et les résultats obtenus.

**TABLEAU I**

| Caractéristiques des catalyseurs activés | | | | | | |
|---|---|---|---|---|---|---|
| Catalyseur n° | Surface BET $(m^2/g)$ | ANALYSE ELEMENTAIRE (% poids) | | | | Perte en vanadium % |
| | | Cr | V | Al | F | |
| *Massiques* | | | | | | |
| 1 | 43,4 | 45,9 | 7,1 | 0 | 19,7 | 7 |
| 2 | 50,5 | 47,6 | 2,4 | 0 | 35 | 7 |
| 3 | 52,4 | 42,2 | 6 | 0 | 31,2 | 9 |
| 4 Comp. | 56,4 | 49,5 | 6,4 | 0 | 19,2 | 25 |
| 5 | 55,8 | 48,3 | 7,6 | 0 | 18,5 | 10 |
| 6 Comp. | 32 | 0 | 48,1 | 0 | 24 | non mesuré |
| 14 Comp. | 110 | 54 | 0 | 0 | 21 | - |
| *Supportés* | | | | | | |
| 7 | 51,6 | 4 | 3,8 | 26,6 | 60,6 | 5 |
| 8 Comp. | 43 | 5,2 | 1,3 | 26,6 | 62,3 | 40 |
| 9 | 45,9 | 6,1 | 4,8 | 25,3 | 59,2 | 3 |
| 10 | 54,5 | 6,6 | 2,6 | 24,8 | 59,6 | 28 |
| 11 Comp. | 47 | 7,1 | 1,4 | 26,8 | 61,9 | 53 |
| 12 Comp. | 39,5 | 6,3 | 0 | 27,2 | 61,8 | - |
| 13 Comp. | 36,3 | 0 | 4,3 | 27,7 | 62,6 | 3 |

EP 0 657 409 A1

## TABLEAU II

### Fluoration du F133a en F134a à 350°C et pression atmosphérique

### Catalyseurs massiques

| ESSAI | F1 | F2 | F3 | F4 | F5 | F6 | F7 | F8 | F9 | F10 | F11 |
|---|---|---|---|---|---|---|---|---|---|---|---|
| *Conditions opératoires* : | | | | | | | | | | | |
| Catalyseur n° | 1 | 1 | 1 | 2 | 3 | 4 | 5 | 14 | 14 | 14 | 6 |
| Rapport molaire HF/F133a | 4,5 | 4,1 | 4 | 5,2 | 4,2 | 4,1 | 4,2 | 3,9 | 4,2 | 3,8 | 3 |
| Rapport molaire $O_2$/F133a | 0 | 0 | 0,005 | 0 | 0 | 0 | 0 | 0 | 0 | 0,005 | 0 |
| Temps de contact (secondes) | 4 | 2,1 | 0,5 | 3,6 | 4 | 3,9 | 3,8 | 4,2 | 3,9 | 0,6 | 3,5 |
| Age du catalyseur (heures) | 20 | 46 | 133 | 24 | 24 | 25 | 23 | 67 | 340 | 24 | 23 |
| *Résultats* : | | | | | | | | | | | |
| Taux de transformation global du F133a (%) | 21,2 | 21,3 | 20,1 | 22,6 | 21,1 | 22,9 | 21,5 | 20,9 | 15,5 | 15,1 | 3 |
| Sélectivité (% molaire) en : | | | | | | | | | | | |
| - F134a ($CF_3CH_2F$) | 97,1 | 97,5 | 93,3 | 97,4 | 97,4 | 95,8 | 95,9 | 95,7 | 97,1 | 92,3 | 34,8 |
| - F1122 ($CF_2=CHCl$) | 0,9 | 0,9 | 1,6 | 0,9 | 0,7 | 1,3 | 1,4 | 1 | 1 | 1,1 | 4,6 |
| - Série F120 (*) | 1,2 | 0,7 | 3,3 | 0,8 | 0,4 | 0,7 | 0,6 | 1 | 0,6 | 4,8 | 0,3 |
| - F143a ($CF_3CH_3$) | 0,5 | 0,6 | 0,2 | 0,2 | 0,8 | 0,9 | 0,7 | 1,4 | 0,3 | 0,3 | 59,6 |
| - autres | 0,3 | 0,3 | 1,4 | 0,7 | 0,7 | 1,3 | 1,4 | 0,9 | 1 | 1,5 | 0,7 |

* Ensemble des hydrogénopentahaloéthanes

EP 0 657 409 A1

## TABLEAU III

*Fluoration du F133a en F134a à 350°C et pression atmosphérique*

*Catalyseurs supportés*

| ESSAI | F12 | F13 | F14 | F15 | F16 | F17 | F18 | F19 | F20 | F21 |
|---|---|---|---|---|---|---|---|---|---|---|
| *Conditions opératoires* : | | | | | | | | | | |
| Catalyseur n° | 7 | 7 | 8 | 9 | 9 | 10 | 12 | 12 | 12 | 13 |
| Rapport molaire HF/F133a | 4,5 | 4,1 | 4,1 | 4 | 4 | 4,1 | 3,8 | 3,9 | 4 | 4 |
| Rapport molaire $O_2$/F133a | 0 | 0,005 | 0 | 0 | 0 | 0 | 0 | 0 | 0,005 | 0 |
| Temps de contact (secondes) | 4 | 0,5 | 4 | 3,9 | 4 | 3,9 | 4,1 | 4,2 | 0,5 | 4 |
| Age du catalyseur (heures) | 20 | 24 | 43 | 24 | 250 | 24 | 24 | 151 | 23 | 19 |
| *Résultats* : | | | | | | | | | | |
| Taux de transformation global du F133a (%) | 19,3 | 15,2 | 21,8 | 20,5 | 20,1 | 20,9 | 20,7 | 15,2 | 12,3 | 3 |
| Sélectivité (% molaire) en : | | | | | | | | | | |
| - F134a ($CF_3CH_2F$) | 98,4 | 96,7 | 98,1 | 98,2 | 98,1 | 97,4 | 97,9 | 98 | 96,6 | 78,4 |
| - F1122 ($CF_2$=CHCl) | 0,7 | 1 | 0,9 | 0,6 | 0,7 | 1,2 | 1,3 | 0,7 | 0,9 | 11,7 |
| - Série F120 (*) | 0,5 | 1,4 | 0,5 | 0,8 | 0,9 | 0,6 | 0,3 | 0,3 | 1,6 | 9,3 |
| - F143a ($CF_3CH_3$) | 0,4 | 0,5 | 0,5 | 0,4 | 0,3 | 0,3 | 0,4 | 0,3 | 0,6 | 0,3 |
| - autres | 0 | 0,4 | 0 | 0 | 0 | 0,5 | 0,1 | 0,7 | 0,3 | 0,3 |

* Ensemble des hydrogénopentahaloéthanes

En ce qui concerne les catalyseurs massiques, les résultats obtenus dans les essais F1 à F3 et F7 et plus particulièrement celui à faible temps de contact (essai F3), traduisent la meilleure activité des catalyseurs mixtes $Cr^{III}$-$V^{III}$ par rapport à l'oxyde de chrome$^{III}$ seul (essais F8 à F10) ou à l'oxyde vanadium $^V$ seul (essai F11). Les essais F1 à F5 montrent que les modes de préparation: voie solgel (catalyseur 1 - essais F1 à F3) et voie oxalate (catalyseurs 2 et 3 - essais F4 et F5) conduisent à des performances catalytiques comparables.

L'examen du Tableau I montre que les pertes en vanadium durant l'activation du catalyseur sont faibles lorsque l'on utilise des précurseurs de chrome et de vanadium à l'état d'oxydation trois (catalyseurs 1 à 3). Les pertes durant l'activation sont plus importantes lorsqu'on utilise un précurseur de vanadium à un état d'oxydation supérieur à trois (catalyseur 4), à moins de procéder à une calcination réductrice avant l'activation (catalyseur 5).

Dans le cas des catalyseurs supportés, les résultats obtenus dans les essais F15 et F16 montrent également la meilleure stabilité des catalyseurs mixtes $Cr^{III}$-$V^{III}$ par rapport à $Cr_2O_3$ seul (essais F18 et F19).

Le catalyseur mixte ex $Cr^{VI}$ et $V^{III}$ supporté sur $AlF_3$ (catalyseur 8) présente une bonne activité (essai F14), mais les pertes en vanadium durant la phase d'activation sont considérables (40 %). Ces pertes sont apparemment dues à une réaction d'oxydo-réduction entre le chrome $^{VI}$ et le vanadium $^{III}$.

## Revendications

1. Procédé de fluoration catalytique d'hydrocarbures halogénés en phase gazeuse au moyen d'acide fluorhydrique, caractérisé en ce qu'on utilise un catalyseur mixte à base de dérivés du chrome et du vanadium, ces deux éléments étant majoritairement à l'état d'oxydation III.

2. Procédé selon la revendication 1, dans lequel le chrome et le vanadium sont à l'état d'oxydation III pour plus de 90 %.

3. Procédé selon la revendication 1 ou 2, dans lequel le rapport atomique V/Cr est compris entre 0,1 et 3,5, de préférence entre 0,15 et 3.

4. Procédé selon l'une des revendications 1 à 3, dans lequel le catalyseur mixte Cr-V est un catalyseur massique.

5. Procédé selon la revendication 4, dans lequel le catalyseur massique est obtenu par calcination entre 200 et 600°C d'un précipité ou gel de composés du chrome $^{III}$ et du vanadium $^{III}$ ou par décomposition thermique d'oxalates de chrome $^{III}$ et de vanadium $^{III}$.

6. Procédé selon la revendication 4 ou 5, dans lequel le catalyseur massique est préparé à partir de chlorure de vanadium $^{III}$ et d'un sulfate, acétate ou nitrate de chrome.

7. Procédé selon l'une des revendications 1 à 3, dans lequel les composés du chrome et du vanadium sont déposés sur un support.

8. Procédé selon la revendication 7, dans lequel le support est le trifluorure d'aluminium ou un oxyde métallique comme l'alumine, la magnésie et la zircone.

9. Procédé selon la revendication 7 ou 8, dans lequel le catalyseur supporté est préparé à partir de chlorure de vanadium $^{III}$ et de chlorure de chrome $^{III}$ ou d'anhydride chromique.

10. Application du procédé selon l'une des revendications 1 à 9 à la fluoration du 1-chloro-2,2,2-trifluoroéthane en 1,1,1,2-tétrafluoroéthane.

**Office européen des brevets**

# RAPPORT DE RECHERCHE EUROPEENNE

Numero de la demande

EP 94 40 2780

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | EP-A-0 295 885 (KAISER ALUMINUM & CHEMICAL CORPORATION) <br> * revendications * <br> ----- | 1 | C07C17/20 <br> C07C19/08 |

**DOMAINES TECHNIQUES RECHERCHES (Int.Cl.6)**

C07C

Le présent rapport a été établi pour toutes les revendications

| Lieu de la recherche | Date d'achèvement de la recherche | Examinateur |
|---|---|---|
| LA HAYE | 7 Mars 1995 | Bonnevalle, E |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)